# EUROPÄISCHE PATENTSCHRIFT

(11) **EP 1 816 986 B1**
(45) Veröffentlichungstag und Bekanntmachung des Hinweises auf die Patenterteilung: **19.03.2008**
(21) Anmeldenummer: 05808159.7
(22) Anmeldetag: 09.11.2005
(51) Int. Cl.: A61F 2/34

(54) **Selbstschneidender Einschraubkörper**
Self-tapping screw-in body
Corps autoteraudeur à insertion par vissage

(30) Priorität: 09.11.2004 DE 102004053944; 30.11.2004 DE 102004057709
(43) Veröffentlichungstag der Anmeldung: 15.08.2007
(73) Patentinhaber: Hörmansdörfer, Gerd, 31303 Burgdorf-Beinhorn (DE)
(72) Erfinder: Hörmansdörfer, Gerd, 31303 Burgdorf-Beinhorn (DE)
(74) Vertreter: Siekmann, Gunnar
(86) Internationale Anmeldenummer: PCT/DE2005/002014
(87) Internationale Veröffentlichungsnummer: WO 2006/050708

(56) Entgegenhaltungen:
- EP-A- 0 358 345
- EP-A- 0 622 058
- WO-A-97/39702
- DE-A- 3 602 081
- DE-C- 3 535 959
- DE-U- 9 402 828
- FR-A- 2 622 432

## Beschreibung

Die Erfindung betrifft einen Einschraubkörper mit einem selbstschneidenden Gewinde, wobei eine umlaufende Gewinderippe durch mindestens eine Spannut in Gewindezähne unterteilt ist, und mit einer mindestens in einem Teilbereich der Gewindeerstreckung liegenden nicht-linearen Mantelfläche, wobei das Gewindezahnprofil in Vorschubrichtung gekippt ist.

Gewinde sind als konstruktive Elemente des allgemeinen Maschinenbaus weit verbreitet. Gewöhnlich sind Gewinde zylinderförmig gestaltet. Daneben sind auch konische Gewinde z. B. für Ölfeldrohre in Gebrauch. Eine große Anzahl verschiedener Gewindeprofile sind bekannt und in Normen festgelegt. Üblicherweise ist das Gewindeprofil an einem Werkstück unveränderlich, das heißt, daß das Gewindeprofil am Gewindeanfang identisch mit dem am Gewindeende ist. Es sind jedoch Ausnahmen vorstellbar, bei denen eine zumindest in einem Teilbereich des Gewindes fließend sich ändernde Formgestalt des aus Gewinderille und Gewindezahn gebildeten Gewindeprofils von Vorteil sein könnte, z. B. um das Einführen eines Schraubengewindes in ein Mutterngewinde zu erleichtern.

Spezielle geometrische Verhältnisse in Bezug auf das Gewinde bestehen jedoch vor allem bei Gewinden auf gekrümmten Flächen, wie sie insbesondere bei einschraubbaren künstlichen Hüftgelenkpfannen vorkommen. Hier sind hinsichtlich der Mantelform des Schalenkörpers z. B. hypo-, hemi-, oder hypersphärische, konischsphärische, parabolische toroidische, elliptische und ähnliche Geometrien bekannt. Bei spanenden Herstellungsverfahren für die Herstellung derartiger Schraubpfannen ergeben sich teilweise zwangsläufig fließend sich verändernde Verzerrungen des Gewindeprofils, welche in den meisten Fällen weder beabsichtigt noch erwünscht sind. Insbesondere bei der Benutzung von Gewindezähnen mit unsymmetrischen Flanken (den jeweiligen Seitenwinkeln des Gewindezahns) ergibt sich das Phänomen, daß je nach Kipprichtung des resultierenden Gewindezahns die Zahnhöhe vom Pfannenäquator in Richtung zum Pfannenpol hin fließend zu- bzw. abnimmt, woraus sich dann am polnahen Gewindeanfang entweder viel zu große oder nahezu verkümmerte Gewindezähne ergeben. Im ersten Fall führen die extrem großen Gewindezähne dazu, daß zum Einschrauben der Pfanne sehr hohe Kräfte erforderlich werden, bzw. das Implantat nicht bis zum vollständigen Knochenkontakt eingeschraubt werden kann. Im zweiten Fall wird lediglich eine sehr dürftige Primärfixation zu erreichen sein. In beiden Fällen besteht die Gefahr des Auslockerns des Implantats, welches als Konsequenz eine nochmalige Operation des Patienten bedeuten würde.

Ein weiteres Problem besteht bei Einschraubkörpern mit gekrümmter Mantelfläche darin, daß sich die einzelnen Gewindezähne beim Einschrauben in eine entsprechend ausgeraffelte Kavität nicht - wie häufig angenommen - gleichzeitig oder gar mit dem in Einschraubrichtung vorn liegenden Gewindeanfang zuerst eingraben. Es ist vielmehr so, daß sich z. B. bei sphärischen Hüftgelenkpfannen mit vier Gewinderippenumläufen der vom Äquator aus gesehen zweite Umlauf zuerst eingräbt, gefolgt vom ersten und dritten Umlauf. Erst danach, wenn sich die genannten drei Umläufe bereits weitgehend eingegraben haben, kommt der vierte Umlauf in Kontakt mit der Kavität. Weil nun die Zähne des ersten bis dritten Umlaufs in der geschnittenen Rille zwangsweise geführt sind, unterliegt der vierte Gewindeumlauf hinsichtlich seiner Eindringrichtung diesem Zwang. Bei einem gekippten Gewindezahnprofil entsteht dann ein Schieben welches auf eine Seite des Gewindezahnprofils verlagert ist.

Aus Rechenmodellen mit finiten Elementen ist bekannt, dass sich eine günstigere Lastübertragung zwischen Implantat und knöchernem Lager durch ein Überkippen des Gewindezahns zum Pfannenpol hin erzielen läßt. Dies resultiert aus der räumlichen Lage des Hauptlastvektors im Bewegungszyklus beim Gehen während des Aufsetzens des Hackens und der relativen Position der implantierten Hüftgelenkpfanne. Wenn das Gewindezahnprofil nun entsprechend in Richtung zum Pfannenpol gekippt ist und die Drallrichtung der Spannut wie üblich der Drallrichtung des Gewindes entspricht, kann die einseitig zwischen Gewindezahn und Spannut in Richtung zum Pfannenpol gebildete Schneidkante keine Wirkung entfalten. Ein unerwünscht hoher Wert des Einschraubdrehmoments ist die Folge dieses Problems.

Der oben geschilderte Sachverhalt ist zumindest teilweise Gegenstand der Europäischen Patentschrift EP 0 898 470 und daher im offengelegten Rahmen allgemein bekannt. In dieser Schrift wird vorgeschlagen, zur Erzielung eines bestimmten Gewindeprofilverlaufs das Gewinde mit einer entsprechend angepaßten Variation der Steigung so auszubilden, daß für sämtliche Zähne des Gewinderippenzuges eine identische Schubrichtung während des Einschraubvorgangs resultiert. Diese Gestaltung wird im Bereich der Medizintechnik bereits bei künstlichen Hüftgelenkpfannen mit Erfolg benutzt. Dabei werden die Schutzrechte der zugehörigen Patentfamilie von einem Hersteller exklusiv genutzt. Allerdings ist für die drehtechnische Herstellung eines derartigen Einschraubkörpers eine Drehmaschine erforderlich, deren CNC-Steuerung die Programmierung einer variablen Gewindesteigung zuläßt.

Es bestand daher die Aufgabe zur Schaffung von spanend auch auf älteren oder nicht entsprechend ausgerüsteten CNC-Maschinen herstellbaren, bzw. alternativen Einschraubkörpern (z.B. in Gestalt von Hüftgelenkpfannen) mit einem mindestens in einem Teilbereich auf einer gekrümmten oder abgewinkelten Mantelfläche liegenden Gewinde mit unsymmetrischen Teilflankenwinkeln des Gewindezahns und einem gegenüber herkömmlichen Einschraubkörpern reduzierten Einschraubdrehmoment.

Bei einem Einschraubkörper mit einem selbstschneidenden Gewinde, wobei die umlaufende Gewinderippe durch mindestens eine Spannut in Gewindezähne unterteilt ist, und mit einer mindestens in einem Teilbereich der Gewindeerstreckung liegenden nicht-linearen, insbesondere gekrümmten und/oder geknickten Mantelfläche, wobei das Gewindezahnprofil in Vorschubrichtung gekippt ist, wird die Aufgabe erfindungsgemäß dadurch gelöst, daß die Spannut die Gewindezähne mit einem Winkel schneidet, welcher entgegengesetzt zur Richtung des Steigungswinkels des Gewindes verläuft.

Bei dem erfindungsgemäßen Einschraubkörper ist die zwischen Spannut und Gewindezahn gebildete Schneidkante auf die der Vorschubrichtung entgegengesetzte Seite des Gewindezahns verlagert, bzw. verläuft die Schrägungs- oder Drallrichtung der Spannut entgegengesetzt der Drallrichtung des Gewindes und der Drallwinkel der Spannut ist größer als der Steigungswinkel des Gewindes. Eine derartige Gestaltung ist für bestimmte Anwendungen vorteilhaft, weil dadurch die zum Einschrauben erforderlichen Kräfte ohne nachteilige Auswirkungen auf die sonstigen Eigenschaften merklich reduziert sind.

Bevorzugt verläuft die Spannut in dem Einschraubkörper schräg. In einer anderen bevorzugten Ausführungsform der Erfindung besitzt die Spannut einen Drall. Günstigerweise ist die Schneidkante auf der rückwärtigen, zum Äquator gerichteten Flanke des Einschraubkörpers angeordnet. Weiterhin ist es günstig, daß der Drallwinkel bzw. der Schrägungswinkel größer ist als der Steigungswinkel des Gewindes. In einer anderen Weiterbildung der Erfindung ist der Einschraubkörper als Implantat und in einer weiteren bevorzugten Ausführungsform als künstliche Hüftgelenkpfanne realisiert.

Im Bereich der Technik sind rechts gedrallte Gewinde Standard. Links gedrallte Gewinde sind besonderen Anwendungen vorbehalten, z. B. wenn bei einer entsprechend gerichteten Torsionsbeanpruchung eine Lockerung der Gewindeverbindung zu erwarten ist. Dem gemäß ist allgemein die Erwartungshaltung tief verwurzelt, eine Schraubverbindung rechtsgängig festzuziehen und linksgängig zu lösen. Alle Schraubdeckel und Schraubkappen sind derart gestaltet, auch z. B. selbstschneidende Schrauben. Bei Einschraubkörpern (z. B. künstlichen Hüftgelenkpfannen) ist das nicht anders. Sind Einschraubkörper durch Spannuten selbstschneidend ausgelegt, so verlaufen diese Spannuten häufig neutral, also mit einem Drallwinkel von null Grad. Der Querschnitt des jeweiligen Gewindezahns bildet dann eine Schneidfläche, welche wegen des Steigungswinkels des Gewindes ganz leicht positiv steht. Manche selbstschneidenden Blechschrauben besitzen leicht links gedrallte oder leicht schräg nach links verlaufende Spannuten, die allerdings lediglich den Steigungswinkel des Gewindes kompensieren und dann mit ihrer Schneidfläche neutral positioniert sind. Links gedrallte Spannuten sind zum Beispiel bei Gewindebohrern bekannt, welche bei Durchgangsgewinden die beim Schneiden anfallenden Späne nach unten hin abtransportieren sollen.

Bei künstlichen Hüftgelenkpfannen sind ausschließlich Rechtsgewinde bekannt. Die Gewindezähne stehen bei den meisten Produkten vertikal zur Pfannenachse. Wenn diese Hüftgelenkpfannen eine gekrümmte oder geknickte Mantelkontur, gerade verlaufende Spannuten und eine konstante Gewindesteigung besitzen, dann sind die Kräfte während des Einschraubvorgangs an der durch die Spannuten an den Gewindezähnen gebildeten Schneidflächen und deren mehr oder weniger neutral stehenden Schneidkanten zumindest theoretisch gleichmäßig verteilt. Wegen der vom Operateur in axialer Richtung während des Einschraubens ausgeübten Andruckkraft (geschätzt: ca. 50 bis 100 N) verlagert sich die Schneidkraft jedoch mehr auf die polwärts liegende Schneidkante des Gewindezahns. Es ist dann überaus sinnvoll, diese Seite mit einer Schneidkante mit positivem Schneidwinkel zu gestalten, weil damit der Einschraubkraftbedarf deutlich reduzierbar ist. Dieses ist in einfacher Weise durch eine rechts gedrallte Spannut mit ausgeprägtem Drallwinkel realisierbar.

Es sind bislang drei künstliche Hüftgelenkpfannen mit linksgerichteter Spannut bei gekrümmter Mantelfläche und neutral stehendem Gewindezahn auf den Markt gekommen. Es sind dies das Modell MC der Firma Brehm, das Modell MT der Firma Zimmer, sowie das Modell PAC der Firma DePuy. Bei der ersteren beträgt der Schrägungswinkel 15°, bei den anderen beiden 10°. Der Spanwinkel der hauptsächlich wirkenden Schneidkante ist dann stumpf (Fachbezeichnung "negativ") und führt daher zu einem ungünstig klemmenden, kömenden und insgesamt nicht akzeptablen Schneidverhalten.

Ganz anders stellt sich die Situation dar, wenn das Gewindezahnprofil bei einer gekrümmten Mantelfläche und konstanter Gewindesteigung zum Pol hin geneigt ist. Der Anmelder hat erkannt, dass dann die polseitige Flanke des Zahns beim Einschraubvorgang quasi ohne Vorschub entlang einer Schnittfläche gleitet, während die äquatorseitige Flanke in das volle Material hinein radial von der Pfannenachse weg vorgeschoben wird. Wird nun die letztere Flanke mittels eines linksgerichteten Dralls oder einer linksgerichteten Schrägung der Spannnut, bzw. mittels eines dem Steigungswinkel des Gewindes entgegengesetzt verlaufenden Drall- oder Schrägungswinkels der Spannt mit Schneidkanten mit positivem oder stark positivem Spanwinkel ausgerüstet, so ist der Einschraubkraftbedarf sehr deutlich reduziert. Der Schnitt ist dann bei Einschraubversuchen in geeignetem Hartschaum glatt, ohne Randausbrüche und ohne das Auftreten von Körnung. Außerdem ist die Taktilianz derart erhöht, daß der Aufsetzpunkt des Implantats nun ohne weiteres erfühlbar ist.

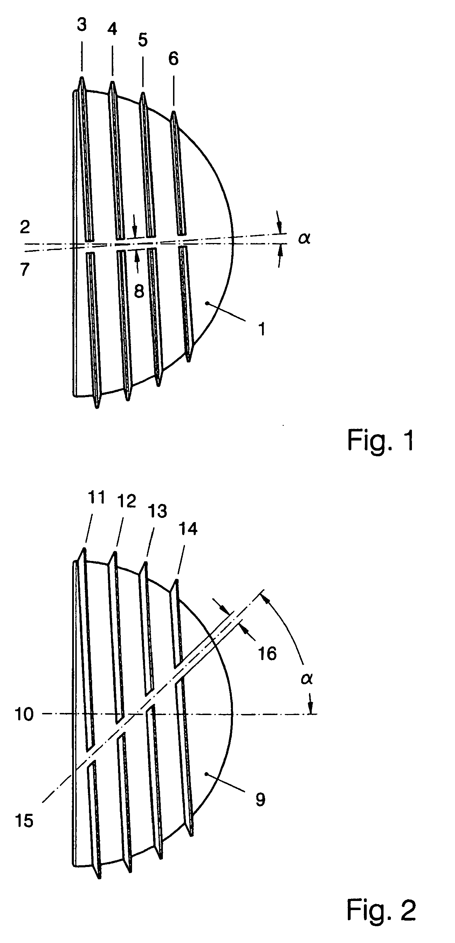

Zum besseren Verständnis soll die Erfindung im folgenden anhand von zwei Zeichnungsfiguren näher erläutert werden. Als schematisches Beispiel eines Einschraubkörpers mit gekrümmter Mantelfläche wurde dabei jeweils auf eine vereinfacht gezeichnete Hüftgelenkpfanne zurückgegriffen. Fig. 1 zeigt eine künstliche Hüftgelenkpfanne mit Gewinde gemäß dem Stand der Technik. In Fig. 2 ist eine erfindungsgemäße Hüftgelenkpfanne mit einem zum Pfannenpol gekippten Gewindezahnprofil und negativem Schrägungswinkel der Spannut dargestellt.

Die Fig. 1 zeigt die schematische Darstellung einer künstlichen Hüftgelenkpfanne 1 gemäß dem Stand der Technik in einem leicht vergrößerten Maßstab. Das gezeigte Beispiel besitzt eine sphärische Kontur des gekrümmten Schalenmantels. Es sind vier umlaufende Zahnrippen 3, 4, 5 und 6 zu sehen, welche durch eine einzige Spannut 8 unterbrochen sind. Die anderen Spannuten wurden aus Vereinfachungsgründen weggelassen. Die Mittelachse der Hüftgelenkpfanne ist durch eine strichpunktierte Linie 2 und die Mitte der Spannut durch eine strichpunktierte Linie 7 angedeutet. Zwischen beiden ist der negative Schrägungswinkel α gebildet. Der Schrägungswinkel entspricht seiner winkelmäßigen Größe nach dem Steigungswinkel des Gewindes, so daß die an den Gewindezähnen durch die Spannut gebildeten Schneidkanten in Bezug auf die Vorschubrichtung beim Einschrauben quer zur Erstreckung der Gewinderippen, und damit neutral stehen.

In der Fig. 2 ist eine der Erfindung gemäße künstliche Hüftgelenkpfanne 9 gleicher Größe und Form dargestellt. Wie zuvor wurde aus Vereinfachungsgründen lediglich eine einzige Spannut 16 eingezeichnet. Auch auf die Darstellung eines Dralls wurde wegen des zeichnerischen Aufwands verzichtet. Das aus vier Umläufen 11, 12, 13 und 14 bestehende Gewinde besitzt ein unsymmetrisches Zahnprofil, welches zum Pfannenpol hin geneigt ist. Diese Kipprichtung bewirkt - wie weiter oben erläutert - eine Vergleichmäßigung der zwischen Implantat und knöchernem Lager zu über tragenden Kräfte. Die Spannut ist in ihrem Winkel demjenigen der Gewindesteigung entgegengerichtet. Mit ihrer strichpunktierten Mittellinie 15 schließt sie gegen die Achse 10 der Hüftgelenkpfanne einen Winkel α von 45° ein. Die an den Gewinderippen gebildeten Schneidkanten liegen nun auf der Rückseite der Gewinderippen auf den zum Pfannenäquator gerichteten Gewindezahnflanken und besitzen wegen den stark schräg verlaufenden Spannuten jeweils einen stark positiven Spanwinkel. Durch die gegenüber dem Stand der Technik günstigere Platzierung der Schneidkanten kommen diese in Verbindung mit dem positiven Spanwinkel voll zur Geltung und bewirken so eine deutliche Senkung des Einschraubdrehmoments und gleichzeitig eine Steigerung der Überdrehreserve.

Mit der Erfindung wird somit ein Einschraubkörper mit selbstschneidendem Gewinde mit einer im Gewindebereich liegenden gekrümmten und/oder geknickten Mantelfläche und in Einschraubrichtung gekipptem Gewindezahnprofil zur Verfügung gestellt, welcher aufgrund der gegenüber der Drallrichtung des Gewindes entgegengesetzten Schrägungs- oder Drallrichtung der Spannut über ein deutlich verbessertes Einschraub- und Überdrehverhalten verfügt und von dem eine Reduzierung der Lockerungsrate erwartet werden kann. Der erfindungsgemäße Einschraubkörper ist ohne technische Probleme und ohne zeitlichen Mehraufwand herstellbar.

## Patentansprüche

1. Einschraubkörper (9) mit einem selbstschneidenden Gewinde (11, 12, 13, 14), wobei eine umlaufende Gewinderippe (11, 12, 13, 14) durch mindestens eine Spannut (16) in Gewindezähne unterteilt ist, und mit einer mindestens in einem Teilbereich der Gewindeerstreckung liegenden nicht-linearen Mantelfläche, wobei das Gewindezahnprofil in Vorschubrichtung gekippt ist,
**dadurch gekennzeichnet,**
**daß** die Spannut (16) die Gewindezähne mit einem Winkel (α) schneidet, welcher entgegengesetzt zur Richtung des Steigungswinkels des Gewindes (11, 12, 13, 14) verläuft.

2. Einschraubkörper (9) gemäß Anspruch 1, **dadurch gekennzeichnet, daß** die Spannut (16) schräg verläuft.

3. Einschraubkörper (9) gemäß Anspruch 1, **dadurch gekennzeichnet, daß** die Spannut (16) einen Drall besitzt.

4. Einschraubkörper (9) gemäß einem der Ansprüche 1 bis 3, **dadurch gekennzeichnet, daß** der Einschraubkörper (9) eine Schneidkante aufweist, die auf der rückwärtigen, zum Äquator gerichteten Flanke des Einschraubkörpers (9) angeordnet ist.

5. Einschraubkörper (9) gemäß einem der Ansprüche 1 bis 4, **dadurch gekennzeichnet, daß** der Drallwinkel größer ist als der Steigungswinkel des Gewindes (11, 12, 13, 14).

6. Einschraubkörper (9) gemäß einem der Ansprüche 1 bis 5, **dadurch gekennzeichnet, daß** der Einschraubkörper (9) als Implantat realisiert ist.

7. Einschraubkörper (9) gemäß Anspruch 6, **dadurch gekennzeichnet, daß** der Einschraubkörper (9) als künstliche Hüftgelenkpfanne realisiert ist.

## Claims

1. A screw-in body (9) comprising a self-tapping thread (11, 12, 13, 14), wherein a circumferential threaded crest (11, 12, 13, 14) is subdivided into threaded teeth by at least one flute (16), and comprising at least one nonlinear circumferential surface which lies in a partial area of the thread extension, wherein the threaded tooth profile is tilted in the advancing direction,
**characterised in**
**that** the flute (16) intersects the threaded teeth at an angle (α) which runs opposite to the direction of the helix angle of the thread (11, 12, 13, 14).

2. The screw-in body (9) according to claim 1, **characterised in that** the flute (16) runs obliquely.

3. The screw-in body (9) according to claim 1, **characterised in that** the flute (16) has a twist.

4. The screw-in body (9) according to any one of claims 1 to 3, **characterised in that** the screw-in body (9) has a cutting edge which is disposed on the rearward flank of the screw-in body (9) directed towards the equator.

5. The screw-in body (9) according to any one of claims 1 to 4, **characterised in that** the angle of twist is greater than the helix angle of the thread (11, 12, 13, 14).

6. The screw-in body (9) according to any one of claims 1 to 5, **characterised in that** the screw-in body (9) can be implemented as an implant.

7. The screw-in body (9) according to claim 6, **characterised in that** the screw-in body (9) is implemented as an artificial acetabulum.

## Revendications

1. Corps à visser (9) avec un filet autotaraudeur (11, 12, 13, 14), une arête de filetage (11, 12, 13, 14) périphérique étant divisée par au moins une goujure (16) en crêtes d'arête, et avec une surface d'enveloppe non linéaire située au moins dans une zone partielle de l'extension du filetage, le profil de la crête d'arête étant basculé en direction d'avance,
**caractérisé en ce que**,
la goujure (16) recoupe les crêtes d'arête sous un angle (α) qui s'écoule à l'opposée de la direction de l'angle d'inclinaison du filetage (11, 12, 13, 14).

2. Corps à visser (9) selon la revendication 1, **caractérisé en ce que** la goujure (16) s'écoule en inclinaison.

3. Corps à visser (9) selon la revendication 1, **caractérisé en ce que** la goujure (16) comporte une torsion.

4. Corps à visser (9) selon l'une quelconque des revendications 1 à 3, **caractérisé en ce que** le corps à visser (9) comporte une arête de coupe qui est disposée sur le flanc arrière, dirigé vers l'équateur du corps à visser (9).

5. Corps à visser (9) selon l'une quelconque des revendications 1 à 4, **caractérisé en ce** l'angle de torsion est supérieur à l'angle d'inclinaison du filetage (11, 12, 13, 14).

6. Corps à visser (9) selon l'une quelconque des revendications 1 à 5, **caractérisé en ce que** le corps à visser (9) est réalisé sous la forme d'un implant.

7. Corps à visser (9) selon la revendication 6, **caractérisé en ce que** le corps à visser (9) est réalisé sous la forme d'une coque acétabulaire pour articulation de la hanche.
